# FASCICULE DE BREVET EUROPEEN

(11) **EP 3 092 041 B1**
(45) Date de publication et mention de la délivrance du brevet: **08.05.2019**
(21) Numéro de dépôt: 15719498.6
(22) Date de dépôt: 06.03.2015
(51) Int. Cl.: A61Q 17/00, A61Q 19/00, A61K 8/98, A61K 35/612, A61K 8/97

(54) **UTILISATION COSMETIQUE D'UN EXTRAIT D'ARTEMIA SALINA POUR PROTEGER LA PEAU DES STRESS THERMIQUES FROIDS**
KOSMETISCHE VERWENDUNG EINES EXTRAKTS AUS ARTEMIA SALINA ZUR HAUTSCHUTZ GEGEN DURCH KÄLTE INDUZIERTE STRESS
COSMETIC USE OF AN EXTRACT OF ARTEMIA SALINA TO PROTECT THE SKIN AGAINST COLD STRESS

(30) Priorité: 08.01.2014 FR 1450132
(43) Date de publication de la demande: 16.11.2016
(73) Titulaire: ISP Investments LLC, Wilmington, DE 19805 (US)
(72) Inventeur: BOTTO, Jean-Marie, F-06560 Valbonne (FR); BUSUTTIL, Valère, F-06100 Nice (FR); CUCUMEL, Karine, F.06650 Opio (FR); ASTLES, Neil, F-06300 Nice (FR); DOMLOGE, Nouha, F-06650 OPIO (FR)
(74) Mandataire: Miquel, Corinne
(86) Numéro de dépôt international: PCT/FR2015/000050
(87) Numéro de publication internationale: WO 2015/107286

(56) Documents cités:
- CN-A- 103 251 652
- FR-A1- 2 810 241
- FR-A1- 2 834 887
- FR-A1- 2 835 743
- JP-A- 2004 238 297
- RU-C1- 2 429 865
- JUN FUJITA: "Cold Shock Response in Mammalian Cells", JOURNAL OF MOLECULAR MICROBIOLOGY AND BIOTECHNOLOGY, HORIZON SCIENTIFIC PRESS, WYMONDHAM, GB, vol. 1, no. 2, 1 janvier 1999 (1999-01-01) , pages 243-255, XP007912820, ISSN: 1464-1801 cité dans la demande

## Description

### Domaine de l'invention

La présente invention se situe dans le domaine de la protection de la peau. L'invention concerne l'utilisation non-thérapeutique d'un extrait *d'Artémia salina* pour protéger la peau du dessèchement et des gerçures liés aux agressions dues aux stress thermiques froids.

L'invention a également pour objet une composition comprenant un milieu physiologiquement acceptable et ledit extrait *d'Artémia salina* pour son utilisation pour protéger la peau des irritations liées aux stress thermiques froids.

### Arrière plan de l'invention

La fonction principale de l'épiderme est d'assurer une barrière protectrice entre l'organisme et l'environnement extérieur. La peau, en contact direct avec l'environnement extérieur, est exposée à de nombreuses variations des conditions environnementales. Selon la nature et l'ampleur des variations, celles-ci peuvent provoquer une agression, responsable d'un stress d'ordre physique, chimique ou biologique. Les principaux stress sont les stress thermiques, l'exposition aux radicaux libres de l'oxygène, les ultraviolets, les infrarouges, les métaux lourds, les chocs osmotiques, les chocs de pression, mais aussi les états pathologiques tels fièvre, inflammation, infection virale...

On définit un stress thermique comme un stress affectant le fonctionnement optimal de l'organisme et qui dépasse les mécanismes physiologiques de thermorégulation du corps. Un stress thermique se produit suite à un abaissement (stress thermique froid) ou une élévation de température (stress thermique chaud), d'origine environnementale ou interne. Une déviation de quelques degrés de cette température optimale peut avoir des conséquences importantes.

Un choc thermique est défini comme un stress provoqué par une variation de température brusque et importante (variation de température d'au moins 10°C).

La peau peut être exposée à des stress thermiques dans de nombreuses situations physiologiques ou accidentelles. La température moyenne de la peau peut être estimée comme une valeur constante qui est d'environ 33°C en fonctionnement optimal, et peut varier rapidement en fonction de l'exposition à l'environnement, par exemple elle tombe à 27°C après immersion dans un bain à 24°C (Marino et Booth, 1998) et peut remonter au-delà de 37°C suite à l'exposition au soleil.

Parmi les stress thermiques froids on peut distinguer l'hypothermie importante, dans laquelle la température des tissus et organes descend en dessous de 25°C (comme celle subie dans l'exposition à des températures froides extrêmes ou bien dans certaines techniques médicales pour préserver les organes et les tissus de l'hypoxie), et l'hypothermie modérée (température comprise entre 25°C et 35°C) comme par exemple l'exposition répétée à l'air conditionné lors des déplacements quotidiens entre pièces intérieures et l'extérieur en été comme en hiver, ou encore la prise de bains.

La peau est fréquemment soumise à des stress thermiques froids modérés suivis de réchauffements successifs. Parmi les effets physiologiques cellulaires de l'exposition au froid on peut citer (Fujita et al.) :
- Augmentation de la dénaturation et désagrégation de protéines
- Ralentissement de la progression à travers le cycle cellulaire, la phase G1 étant en général la plus sensible
- Inhibition de la transcription et de la traduction, ce qui conduit à une réduction générale de la synthèse protéique
- Perturbation des éléments du cytosquelette cellulaire
- Modifications de la perméabilité de la membrane qui entraîne une augmentation cytosolique Na + et les ions H +.

En outre, l'exposition répétée de la peau a des variations de températures répétées, peut provoquer des effets délétères sur le phénotype cellulaire ou encore sur la structure et les compositions lipidiques des membranes cellulaires, altérant ainsi la fonction barrière de la peau et entrainant des irritations et des dessèchements ou encore des gerçures. Ces effets des variations de température et des stress thermiques froids accélèrent le processus de vieillissement de la peau.

La résistance aux stress thermiques est permise par la mise en place d'une réponse cellulaire spécifique. Dans le cas d'un stress thermique froid, la cellule met en place un système de protection en induisant la transcription d'une famille de gènes spécifiques qui aboutit à la synthèse et l'accumulation intracellulaire de protéines de réponse au stress thermique froid ou « CSP » (Cold Shock Proteins) celles-ci étant différentes des protéines de réponse au stress thermique chaud ou « HSP » (Heat Shock Proteins).

Parmi les CSP, la protéine de liaison à l'ARN inductible par le Froid, ou CIRBP (pour Cold-inducible RNA-binding protein) est une protéine codée, chez l'homme, par le gène de la CIRBP. Ces protéines sont exprimées constitutivement ainsi qu'après l'exposition au froid. Les CIRBP jouent un rôle critique dans le contrôle de la réponse cellulaire face à une variété de stress cellulaires, y compris la lumière ultraviolette de courte longueur d'onde, l'hypoxie, et l'hypothermie. Leur expression est rapidement et significativement accrue pendant une hypothermie modérée (Fujita et al., J. Mol. Microbiol. Biotechnol., 1999 ; Larry et al., J. Appl. Physiol., 2002) parmi les autres rôles des CIRBP :
- Inhibition de la dégradation de l'ARN
- Augmentation de la transcription de gènes cibles spécifiques via des éléments de la région promotrice de ces gènes,
- L'épissage alternatif du pré-ARNm

Les propriétés particulières des CIRBP en font donc des marqueurs biologiques intéressants de la réaction de l'organisme aux chocs de température.

Les extraits *d'Artémia salina* sont déjà utilisés en cosmétique. Par exemple, le document FR 2 817 748 décrit un extrait *d'Artémia salina* pour prévenir le vieillissement de la peau dû aux agressions UV, le document FR 2 835 743 décrit un extrait *d'Artémia salina* pour limiter les effets secondaires des rétinoïdes ou encore la demande WO 1999038483 qui décrit un produit cosmétique a base d'extraits d*'Artémia salina* pour la régénération et la stimulation des cellules de la peau.

Le document JP2004 238297 décrit l'utilisation de cystes *d'Artémia salina* pour protéger la peau contre les agressions de l'environnement externe tels que les produits chimiques, les rayons ultraviolets, la sécheresse, les fortes températures ainsi que les basses températures, permettant ainsi d'augmenter la fonction barrière de la peau.

Les inventeurs ont mis en évidence, que l'application d'une quantité efficace d'un extrait d*'Artémia salina* permet de protéger les cellules, des stress thermiques froids.

L'invention et les avantages qui en découlent seront mieux compris à la lecture de la description.

### Exposé de l'invention

La présente invention se rapporte à l'utilisation cosmétique et/ou dermatologique, d'un extrait d*'Artémia salina,* pour protéger la peau des agressions dues aux stress thermiques froids.

Le terme «peau» selon l'invention englobe l'ensemble des annexes kératiniques présentes à la surface du corps, en particulier les poils, les cils, les sourcils, les ongles et les cheveux.

Ainsi, on entend par «protéger la peau des agressions dues aux variations de température» au sens de la présente invention, un extrait *d'Artémia salina* utilisé pour diminuer ou prévenir les dommages et irritations de la peau, causés par les stress thermiques froids. Ces agressions répétées de la peau sont considérées comme inesthétiques et souvent associées à l'accélération du vieillissement de la peau.

L'invention concerne plus précisément l'utilisation cosmétique et/ou dermatologique, d'un extrait d*'Artémia salina,* pour maintenir un taux physiologique de CIRBP dans les cellules de la peau exposées a un stress thermique froid.

En effet, l*'Artémia salina* est un petit crustacé qui vit dans les eaux saumâtres. Lorsque les conditions environnementales deviennent difficiles (déshydratation, augmentation- de la teneur en minéraux), l'Artémia s'encapsule et entre dans une phase de dormance, dans laquelle il peut rester plusieurs années. Lorsque les conditions redeviennent favorables, l'Artémia se réhydrate et reprend son cycle de développement harmonieusement.

En raison de l'adaptation à son biotope hyperminéralisé, l*'Artémia salina* a su développer d'importantes facultés d'adaptation : il synthétise le GP4G (diguanosine tétraphostate), molécule énergétique précurseur d'ATP, de GTP et activateur des protéines G. Il possède des cellules spécialisées dans la régulation de l'osmolarité, corollaire du bon équilibre hydrominéral du plancton.

Dans un mode de réalisation particulier, l'extrait d'Artémia contient entre 120 et 195 mg/litre de diguanosine tetraphosphate, de préférence au moins 150 mg/litre de diguanosine tetraphosphate.

Dans la suite de l'exposé, on utilisera indifféremment les termes « agent actif » et « extrait d*'Artémia salina*».

On entend par « application topique », le fait d'appliquer ou d'étaler l'agent actif selon l'invention, ou une composition le contenant, à la surface de la peau ou d'une muqueuse.

On entend par « physiologiquement acceptable », que l'agent actif selon l'invention, ou une composition le contenant, est approprié pour entrer en contact avec la peau ou une muqueuse sans provoquer de réactions de toxicité ou d'intolérance.

On entend par « agressions dues au stress thermique » une exposition au froid, ayant pour conséquence de fragiliser, assécher, irriter ou encore gercer la zone de la peau ayant subi l'agression.

Dans des modes de réalisation particuliers, l'extrait d'Artémia selon l'invention peut être dilué dans un ou plusieurs solvants physiologiquement acceptables, choisi parmi l'eau, le glycérol, l'éthanol, le propanediol, le butylène glycol, le dipropylène glycol, les diglycols éthoxylés ou propoxylés, les polyols cycliques ou tout mélange de ces solvants.

Avantageusement, l'extrait d'Artémia est présent à une concentration comprise entre 0,0001 % et 20 % du poids total de la composition, préférentiellement à une concentration comprise entre 0,01 % et 10% du poids total de la composition, et plus préférentiellement encore à une concentration comprise entre 0,05 % et 5 % du poids total de la composition, dans un milieu physiologiquement acceptable.

Selon un autre mode de réalisation avantageux de l'invention, l'agent actif peut être encapsulé ou inclus dans un vecteur cosmétique tels que les liposomes ou toute autre microcapsule utilisée dans le domaine de la cosmétique ou adsorbé sur des polymères organiques poudreux, des supports minéraux comme les talcs et bentonites.

Les compositions pour la mise en oeuvre de l'invention pourront notamment se présenter sous forme d'une solution aqueuse, hydro-alcoolique ou huileuse; d'une émulsion huile-dans-eau, eau-dans-huile ou émulsions multiples ; elles aussi peuvent se présenter sous forme de suspensions, ou encore poudres, adaptées à une application sur la peau.

Ces compositions peuvent être plus ou moins fluides et avoir l'aspect d'une crème, d'une lotion, d'un lait, d'un sérum, d'une pommade, d'un gel, d'une pâte ou d'une mousse. Elles peuvent aussi se présenter sous forme solide, comme un stick ou être appliquées sur la peau sous forme d'aérosol.

Ces compositions peuvent comprendre, en outre, tout additif communément utilisé dans le domaine d'application envisagé ainsi que les adjuvants nécessaires à leur formulation, tels que des solvants, des épaississants, des diluants, des antioxydants, des colorants, des filtres solaires, des agents auto-bronzants, des pigments, des charges, des conservateurs, des parfums, des absorbeurs d'odeur, des actifs cosmétiques ou pharmaceutiques, des huiles essentielles, des vitamines, des acides gras essentiels, des tensioactifs, des polymères filmogènes, etc.

Dans tous les cas, l'homme de métier veillera à ce que ces adjuvants ainsi que leurs proportions soient choisis de telle manière à ne pas nuire aux propriétés avantageuses recherchées de la composition selon l'invention. Ces adjuvants peuvent, par exemple, correspondre à 0,01 à 20 % du poids total de la composition. Lorsque la composition de l'invention est une émulsion, la phase grasse peut représenter de 5 à 80 % en poids et de préférence de 5 à 50 % en poids par rapport au poids total de la composition. Les émulsionnants et co-émulsionnants utilisés dans la composition seront choisis parmi ceux classiquement utilisés dans le domaine considéré. Par exemple, ils peuvent être utilisés en une proportion allant de 0,3 à 30 % en poids, par rapport au poids total de la composition.

Avantageusement, la composition utilisable pour réaliser l'invention peut comprendre, outre l'agent actif selon l'invention, au moins un autre agent actif présentant des effets cosmétiques similaires et/ou complémentaires à ceux de l'invention. Selon l'invention, cet agent actif sera défini comme un « agent actif additionnel ».

Par exemple, le ou les agents actifs additionnels peuvent être choisi parmi : les agents anti-âges, raffermissants, éclaircissants, hydratants, drainants, favorisant la microcirculation, agents pharmaceutiques, exfoliants, desquamants, stimulant la matrice extracellulaire, activant le métabolisme énergétique, antibactériens, antifongiques, apaisants, anti-radicalaires, anti-UV, anti-acnés, anti-inflammatoires, anesthésiques, procurant une sensation de chaleur, procurant une sensation de fraicheur, amincissants.

De tels agents additionnels peuvent être choisis dans les groupes comprenant :
- la vitamine A et notamment l'acide rétinoïque, le rétinol, le rétinol proprionate, le rétinol palmitate,
- la vitamine B3 et plus particulièrement le niacinamide, le nicotinate de tocophérol,
- la vitamine B5, la vitamine B6, la vitamine B12, le panthénol,
- la vitamine C, notamment l'acide ascorbique, l'ascorbyl glucoside, l'ascorbyl tetrapalmitate, magnesium et sodium ascorbyl phosphate,
- les vitamines E, F, H, K, PP, le coenzyme Q10,
- les inhibiteurs de métalloprotéinase, ou un activateur des TIMP,
- la DHEA, ses précurseurs et dérivés,
- les acides aminés tels que l'arginine, ornithine, hydroxyproline, hydroxyproline dipalmitate, palmitoylglycine, hydroxylysine,méthionine et ses dérivés, composés acides aminés N-acyl,
- les peptides naturels ou de synthèse, incluant les, di-, tri-, tetra-, penta- et hexapeptides et leurs dérivés lipophiles, isomères et complexés avec d'autres espèces telles qu'un ion métal (e.g. cuivre, zinc, manganèse, magnésium, et autres). Par exemple les peptides commercialement connus sous le nom MATRIXYL®, ARGIRELINE®, COLLAXYL™, PEPTIDE VINCI 02™, CHRONOGEN™, LAMINIXYL IS™, PEPTIDE Q10™,
- les extraits peptidiques végétaux tels que extraits de soja, d'épeautre, de vigne, de colza, de lin, de riz, de maïs, de pois,
- les extraits de levures,
- l'acide déhydroacétique (DHA),
- les phystostérols d'origine synthétique ou naturelle,
- l'acide salicylique et ses dérivés, les alpha- et bêta-hydroxyacides, les silanols,
- les sucres aminés, glucosamine, D-glucosamine, N-acetyl- glucosamine, N-acetyl-D-glucosamine, mannosamine, N-acetyl mannosamine, galactosamine, N-acetyl galactosamine,
- les extraits de polyphénols, isoflavones, flavonoïdes, tels que les extraits de raisin, les extraits de pin, les extraits d'olives,
- les lipides tels que les céramides ou les phospholipides, les huiles d'origine animale, telles que le squalène ou le squalane ; les huiles végétales, telles que l'huile d'amandes douces, de coprah, de ricin, de jojoba, d'olive, de colza, d'arachide, de tournesol, de germes de blé, de germes de maïs, de soja, de coton, de luzerne, de pavot, de potiron, d'onagre, de millet, d'orge, de seigle, de carthame, de passiflore, de noisette, de palme, de noyau d'abricot, d'avocat, de calendula ; les huiles végétales éthoxylées, le beurre de karité,
- Tous écrans UV et filtres solaires,
- l'AMP cyclique et ses dérivés, les agents activateurs de l'enzyme adénylate cyclase et les agents inhibiteurs de l'enzyme phosphodiestérase, l'extrait de centella asiatica, l'asiaticoside et l'acide asiatique, les méthyls xanthines, la théine, la caféine et ses dérivés, la théophylline, la théobromine, la forskoline, l'esculine et l'esculoside, les inhibiteurs d'ACE, le peptide Val-Trp, l'inhibiteurs du neuropeptide Y, l'enkephaline, l'extrait de gingko biloba, l'extrait de dioscorea, la rutine, l'extrait de yerba mate, l'extrait de guarana, les oligosaccharides, les polysaccharides, la carnitine, l'extrait de lierre, l'extrait de fucus, l'extrait hydrolysé de Prunella vulgaris, l'extrait hydrolysé de Celosia cristata, l'extrait d'Anogeissus leiocarpus, l'extrait de feuilles de Manihot utilissima, la palmitoylcarnitine, la carnosine, la taurine, l'extrait de sureau, les extraits d'algue tel que l'extrait de Palmaria Palmata, le peptide de synthèse de séquence Arg-Gly-Ser-NH2, commercialisé sous le nom ATPeptide™.

La composition utilisable selon l'invention sera appliquée par voie, topique externe, et la formulation des compositions sera adaptée par l'homme du métier.

Avantageusement, les compositions selon l'invention se présentent sous une forme adaptée à l'application par voie topique. Ces compositions doivent donc contenir un milieu physiologiquement acceptable, c'est-à-dire compatible avec la peau, et couvrent toutes les formes cosmétiques.

Il est bien évident que l'invention s'adresse aux mammifères en général, et plus particulièrement aux êtres humains.

D'autres avantages et caractéristiques de l'invention apparaîtront mieux à la lecture des exemples donnés à titre illustratif et non limitatif.
Figure 1 : expression de CIRBP après un stress thermique froid dans les cellules traitées ou non traitées par l'extrait d'Artémia.
Figure 2 : évaluation des ARNm de CIRBP après un stress thermique froid dans les cellules traitées ou non traitées par l'extrait d'Artémia.

### Exemple 1 : Préparation d'un extrait d'Artémia salina

50 grammes de cystes *d'Artémia salina,* sont réhydratés de 30 minutes à 6 heures dans 1 litre d'eau distillée, à une température allant de 30 °C à 75 °C, dans un milieu adapté, principalement constitué d'eau, et à un pH compris entre 4 et 7. Ces cystes sont ensuite broyés. L'extrait ainsi obtenu est centrifugé et filtré. L'extrait est enfin stérilisé par filtration stérilisante et chauffage à 65°C.

L'extrait est ensuite traité pour se conformer aux exigences cosmétiques (couleur, odeur, aspect, stérilité...).

Un dosage par Chromatographie en phase liquide à haute performance (HPLC) est réalisé pour confirmer une teneur en diguanosine tétraphosphate supérieure à 150 mg/litre.

### Exemple 2 : expression de CIRBP après un stress thermique froid sur cellules traitées ou non traitées par l'extrait d'Artémia salina selon l'invention

Le but de cette étude est de déterminer l'effet d'un prétraitement avec l'extrait *d'Artémia salina* sur les kératinocytes soumis à un stress hypothermique. En particulier, la protéine CIRBP (Cold-Inducible RNA Binding Protein) a été dosée par Western blot sur des kératinocytes soumis à une hypothermie modérée (32°C), avec ou sans prétraitement par l'extrait d'Artemia salina selon l'exemple 1.

Protocole : Des kératinocytes humains normaux (KHN) issus de peau sont traités par addition dans leur milieu de culture d'un extrait *d'Artémia salina* selon l'exemple 1, à 1% et 3%. Cette application est renouvelée une fois par jour, pendant 48 heures. En parallèle, des cultures de KHN sont maintenues sans traitement, de manière à constituer un contrôle non traité. Cette culture se déroule à 37°C.

Puis, les cultures de KHN prétraitées ou non sont soumises à un stress hypothermique en les plaçant à 32°C pendant 6 heures. A l'issue de cette incubation, les KHN sont lysés afin de procéder à l'analyse de l'expression de CIRBP par Western blot. Cette technique classique d'immuno-transfert comporte les étapes suivantes: saturation de la membrane de transfert par une solution de TBS 1X / lait 5% pendant 1 heure, incubation avec l'anticorps primaire CIRBP (Novusbio), à 4°C pendant la nuit puis, incubation avec l'anticorps secondaire couplé à la péroxidase (Santa Cruz) pendant 1 heure à température ambiante. La luminescence est révélée par l'ajout du substrat (SuperSignal West Dura Extended Duration Substrate, Thermo Scientific) à l'aide d'une caméra (Chemi-Imager system, Alpha Innotech Corporation).

### Résultats :

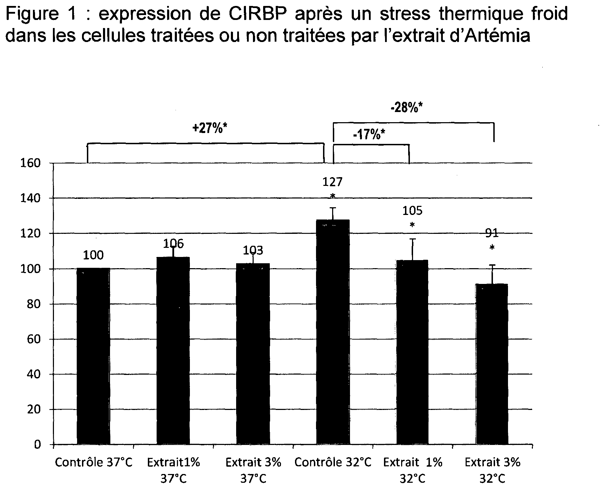

Les résultats, en référence à la figure 1, montrent une augmentation de 27% de l'expression de CIRBP lorsque les cellules sont incubées à 32°C pendant 6 heures, en comparaison avec le contrôle à 37°C et en accord avec les données bibliographiques.

Lorsque les cellules ont été prétraitées par l'extrait *d'Artémia salina* à 1% selon l'exemple 1, à 32°C le taux de CIRBP est diminué significativement, de 17% comparé au contrôle à 32°C. Lorsque les cellules ont été prétraitées par l'extrait d*'Artémia salina* à 3% selon l'exemple 1, à 32°C le taux de CIRBP est diminué significativement, de 28% comparé au contrôle à 32°C.

### Conclusion :

On observe une diminution de l'expression de CIRBP dans les cellules prétraitées par l'extrait *d'Artémia salina* à 1% ou 3% selon l'exemple 1, et soumises à un stress thermique froid, comparé aux cellules contrôle non traitées.

### Exemple 3 : Evaluation des ARNm de CIRBP après un stress thermique froid

Le but de cette étude est de déterminer si les ARNm de CIRBP sont modulés par un traitement par l'extrait *d'Artémia salina* obtenu selon l'exemple 1, préalablement à un stress hypothermique modéré (32°C). Le taux d'ARNm de CIRBP a été évalué par PCR quantitative (Q-PCR).

Protocole : Des kératinocytes humains normaux (KHN) sont traités par l'extrait *d'Artémia salina* à 1% dans le milieu de culture. En parallèle, des cultures de KHN sont maintenues sans traitement, de manière à constituer un contrôle non traité. Cette culture se déroule à 37°C. Puis, les cultures traitées et non traitées sont placées à 32°C pendant 6 heures.

A l'issue de cette incubation, les ARN totaux sont extraits avec le RNeasy mini kit (QIAGEN, 74104) et reverse transcrits avec le High Capacity cDNA reverse-transcription kit contenant des inhibiteurs de RNAses (Applied Biosystems, 4368814). La PCR quantitative est réalisée à l'aide du thermocycler Step One Plus (Applied Biosystems). Les primers et sondes de la cible CIRBP et du contrôle endogène 18S sont issus des Taqman Expression Assays (Applied Biosystems, Hs99999901_s1 pour 18S et CIRBP: Hs00989762_g1), dilués dans de l'eau stérile et du Master Mix (Applied Biosystems).

### Résultats :

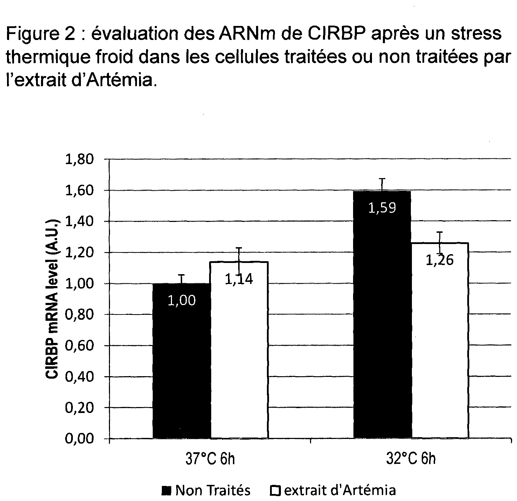

Les résultats, tels que présentés en figure 2, ont montré une augmentation significative de 59% de l'expression des ARNm de CIRBP dans les kératinocytes non traités soumis à une hypothermie modérée (32°C), comparé au contrôle à 37°C.

Dans le cas où les kératinocytes ont été traités par l'extrait *d'Artémia salina* à 1% préalablement à l'exposition au stress hypothermique, leur taux d'ARNm de CIRBP a été augmenté de 12% par rapport aux kératinocytes prétraités par l'extrait à 37°C et le taux d'ARNm de CIRBP était diminué de 33%, en comparaison au kératinocytes contrôle non traités à 32°C.

### Conclusion :

On observe un taux plus faible d'ARNm de CIRBP dans les kératinocytes prétraités par l'extrait *d'Artémia salina* selon l'exemple 1 et soumis à l'hypothermie, comparé aux cellules contrôle non traitées.

### Exemple 4 : Effet de l'extrait de l'exemple 1 sur la perte insensible en eau (TEWL)

### Objectif :

L'étude permet d'évaluer l'efficacité de l'extrait *d'Artemia salina* obtenu selon l'exemple 1, sur la protection de la peau exposée au stress thermique, en particulier sur la perte insensible en eau (TEWL) lors d'un stress chaud/froid. La perte insensible en eau (TEWL) est un paramètre qui reflète indirectement la perméabilité et la fonction barrière de la peau (Toby Mathias et al., 1981). Le stress chaud/froid mime les différences de températures entre l'intérieur et l'extérieur en été comme en hiver.

### Protocole :

L'étude a été conduite sur 19 volontaires, âgés de 21 à 52 ans, dont 9 dans un groupe « mâture » (âges 48 à 57) et 10 dans un groupe «jeune» (âges entre 21 et 34 ans).

Selon les volontaires, la nature des peaux était normale ou sèche, sans pathologie cutanée, et de phototype de 2 à 4.

Le test a été réalisé en double aveugle, contre 1% d'extrait d'Artémia salina selon l'exemple 1, et sa durée a été de 3 semaines.

L'extrait d'*Artemia salina* de l'exemple 1 et le placebo ont été appliqués 2 fois par jour, le matin et le soir, à une concentration de produit sur une zone identique de chaque cuisse.

La quantité de produit appliquée a été de 2 mg/cm².

Procédé de stress thermique : Tout d'abord un coussin chauffant (coussin chauffant électrique WELLYS®) a été appliqué pendant 10 mn sur la cuisse. Immédiatement après, est appliquée une poche de glace pendant 10 mn (poche de glace FirstIce® (EZY WRAP))

La perte insensible en eau (TEWL pour « *Trans Epidermal Water Loss »)* a été évaluée à l'aide de l'évaporimètre Dermalab® (Cortex Technology). La mesure est exprimée en g/m²/h et s'interrompt quand l'écart type est de 0,1 ou après 60 secondes. La mesure a été pratiquée sous des conditions contrôlées de température et d'humidité (21 °C ± 1 et 50 % ± 5).

### Mode opératoire :

1) Mesures basales,
2) Application des produits,
3) Mesures à Jour 0, à Jour 21 avant le stress et Jour 21 après le stress.

Pour analyser les résultats nous avons observé le temps d'obtention d'un plateau de la courbe des mesures de TEWL. Ce temps correspond au temps que la peau met pour récupérer, c'est-à-dire revenir à l'état de base, après un stress thermique. Nous avons appelé ce temps le « temps de récupération ». Ensuite nous avons statistiquement comparé ce temps de récupération pour les côtés traités avec l'extrait selon l'exemple 1, et les côtés traités avec le placebo.

Après le stress des mesures de TEWL on été faite toutes les 2 min

### Résultat :

Nous notons que le plateau de la courbe est atteint plus rapidement pour les zones traitées avec l'extrait d'Artémia que pour les zones traitées au placebo.

Le temps de récupération est de manière significative 27% plus rapide en moyenne pour les zones traitées avec l'extrait d'Artémia que pour les zones traitées au placebo.

### Conclusion :

Les valeurs de TEWL reviennent plus rapidement à la normale après un stress thermique froid-chaud, sur les zones traitées avec l'extrait d'Artémia salina selon l'exemple 1, par rapport au placebo.

Ce résultat montre que l'extrait d'Artémia salina aide la peau à récupérer plus rapidement après un stress thermique chaud/froid.

### Exemple 5 : Préparation de compositions

### 1 Crème protectrice

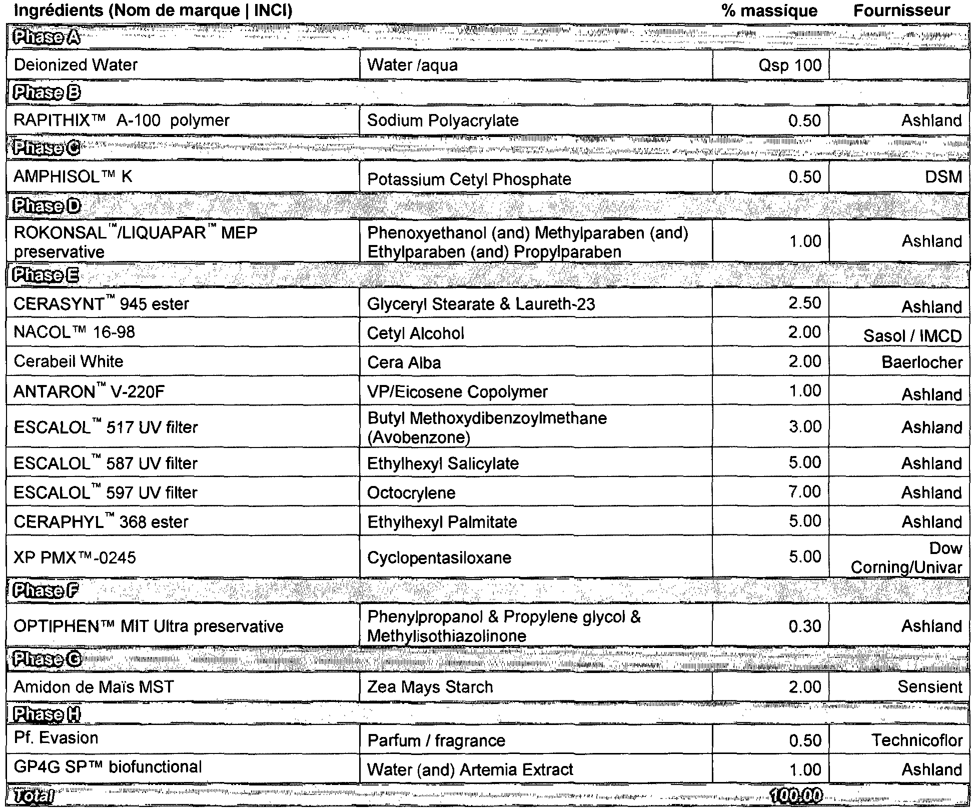

### Mode opératoire

1. Homogénéiser la phase A dans la cuve principale
2. Saupoudrer de la phase B et homogénéiser pendant 10 minutes, puis commence à chauffer à 50 ° C et bien mélanger pendant 30 minutes
3. Refroidir la température et ajouter la phase C. Bien mélanger pour homogénéiser
4. Dans un bécher de côté préparer phase D, chauffer à 55-60 ° C jusqu'à homogénéité et refroidir la température
5. Ajouter les éléments de la phase E un à un dans la cuve principale et bien mélanger entre chaque addition
6. Ajouter la phase D (à température ambiante) dans le vaisseau principal et mélanger jusqu'à homogénéité
7. A 25 ° C, ajouter la phase F et bien mélanger
8. Prémélanger la phase G avant de l'ajouter à la cuve principale
9. Ajouter les éléments de la phase H, un par un et bien mélanger entre chaque addition.
10. Arrêt à 25 ° C

### 2 Crème hydratante pour homme

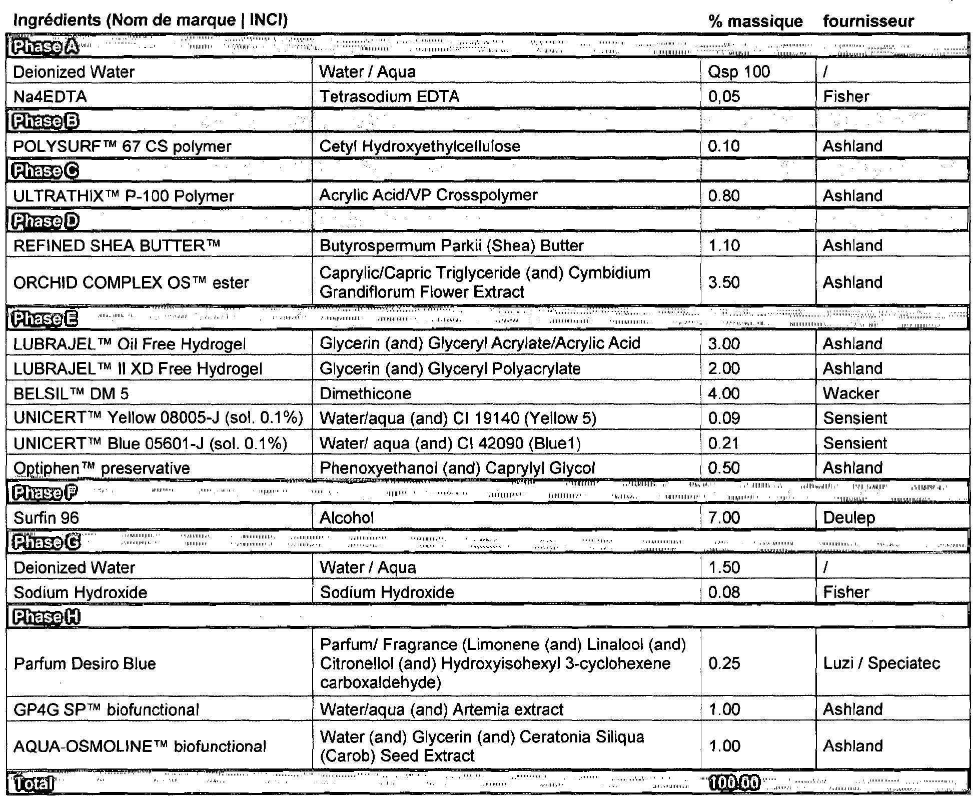

Mode opératoire
1. Homogénéiser la phase A dans la cuve principale
2. Saupoudrer de la phase B et homogénéiser pendant 10 minutes jusqu'à homogénéité
3. Dans un bécher de côté préparer la phase C, homogénéiser jusqu'à l'homogénéité. Saupoudrer dans la phase D et bien mélanger jusqu'à homogénéité
4. A 25 ° C, ajouter la phase C + D dans le vaisseau principal et mélanger jusqu'à homogénéité
5. A 25 ° C, ajouter la phase E dans le vaisseau principal et mélanger jusqu'à homogénéité
6. A 25 ° C, ajouter la phase C dans le vaisseau principal et mélanger jusqu'à homogénéité
7. Arrêt à 25 ° C

### 3 Baume pour les yeux

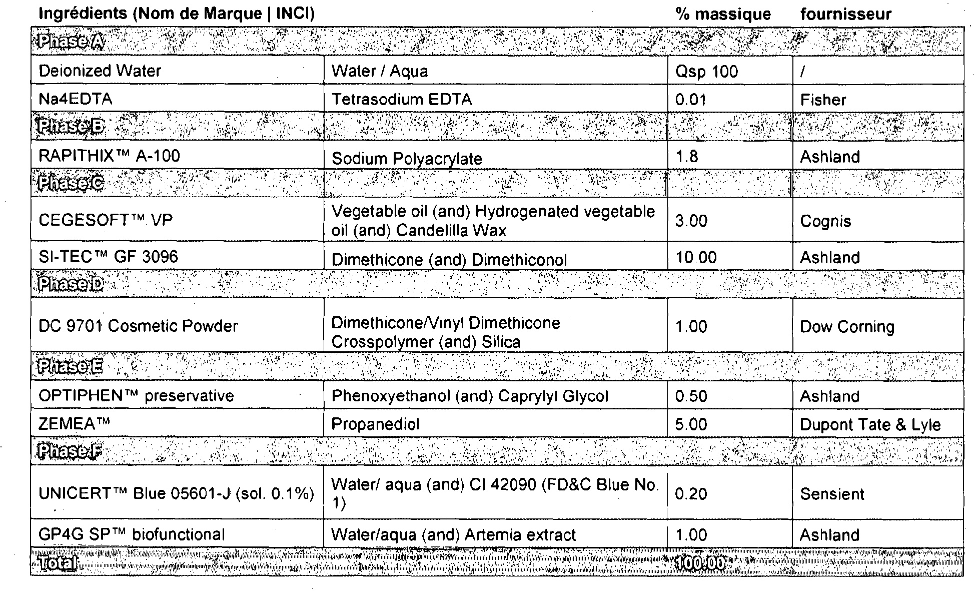

Mode opératoire
1. Ajouter de l'eau dans la cuve principale et commencer à chauffer à 80 ° C.
2. Saupoudrer dans la phase B à 60 ° C et bien mélanger jusqu'à homogénéité.
3. A 80 ° C, ajouter la phase C et bien mélanger et ajouter la phase D
4. Ajouter les ingrédients de la phase E dans un bécher de côté et chauffer à 75-80 ° C.
5. A 80 ° C, ajouter la phase E dans la cuve principale et bien mélanger. L'émulsion doit être homogène.
6. Commencer le refroidissement
7. À 65 ° C, ajouter la phase F et bien mélanger
8. Refroidir jusqu'à 30 ° C et ajouter la phase G puis la phase H en mélangeant bien entre chaque phase jusqu'à homogénéité
9. Arrêt à 25 ° C.

## Revendications

1. Utilisation non thérapeutique pour application topique sur au moins une partie de la peau du corps ou du visage d'une composition comprenant un milieu physiologiquement acceptable et un extrait d'Artemia salina obtenu par le procédé comprenant les étapes suivantes : a) des cystes d'Artemia salina sont réhydratés pendant 30 minutes à 6 heures dans de l'eau distillée, à une température comprise entre 30 °C et 75 °C et à un pH compris entre 4 et 7, b) les cystes sont ensuite broyés et c) l'extrait ainsi obtenu est centrifugé et filtré, pour protéger la peau du dessèchement lié aux agressions dues aux stress thermiques froids en permettant le maintien d'un taux physiologique de CIRP (Cold-inducible RNA Binding Protein).

2. Utilisation selon la revendication 1, dans laquelle ledit extrait d'Artemia salina est dilué dans un ou plusieurs solvants physiologiquement acceptables, choisi parmi l'eau, le glycérol, l'éthanol, le propanediol, le butylène glycol, le dipropylène glycol, les diglycols éthoxylés ou propoxylés, les polyols cycliques ou tout mélange de ces solvants.

3. Utilisation selon l'une des revendications 1 ou 2 dans laquelle ledit extrait d'Artémia salina, est présent à une concentration comprise entre 0,0001 % et 20 % du poids total de la composition, et préférentiellement à une concentration comprise entre 0,05 % et 5 % du poids total de la composition.

4. Utilisation selon l'une des revendications 1 à 3 dans laquelle la composition comprend, en outre, au moins un agent actif additionnel choisi parmi la vitamine A, l'acide rétinoïque, le rétinol, la vitamine B3, la vitamine B5, la vitamine B6, la vitamine B12, la vitamine C, la vitamine E, la vitamine F, la vitamine H, la vitamine K, la vitamine PP, le coenzyme Q10, les inhibiteurs de métalloprotéinase, les acides aminés, la carnitine, la carnosine, la taurine, les peptides naturels ou de synthèse, les extraits peptidiques végétaux, les extraits de levures, les phystostérols d'origine synthétique ou naturelle, l'acide salicylique, les oligosaccharides, les polysaccharides, les sucres aminés, les polyphénols, les flavonoïdes, les lipides, les phospholipides, l'AMP cyclique et ses dérivés, les agents activateurs de l'enzyme adénylate cyclase, les agents inhibiteurs de l'enzyme phosphodiestérase, la théine, la théophylline, la théobromine, la forskoline, l'esculine, les inhibiteurs d'ACE, l'extrait de dioscorea, l'extrait de guarana, l'extrait de lierre, l'extrait de fucus, les extraits d'algues tels que Palmata Palmaria, l'extrait hydrolysé de Prunella vulgaris ou l'extrait de sureau.

5. Composition pour application topique sur au moins une partie de la peau du corps ou du visage, ladite composition comprenant un milieu physiologiquement acceptable et un extrait d'Artemia salina obtenu par le procédé comprenant les étapes suivantes : a) des cystes d'Artemia salina sont réhydratés pendant 30 minutes à 6 heures dans de l'eau distillée, à une température comprise entre 30 °C et 75 °C et à un pH compris entre 4 et 7, b) les cystes sont ensuite broyés et c) l'extrait ainsi obtenu est centrifugé et filtré, pour son utilisation pour protéger la peau des irritations et des gerçures liées aux agressions dues aux stress thermiques froids en permettant le maintien d'un taux physiologique de CIRBP (Cold-inducible RNA-Binding Protein).

## Patentansprüche

1. Nicht-therapeutische Verwendung, zur topischen Anwendung auf mindestens einem Teil der Haut des Körpers oder des Gesichts, einer Zusammensetzung, umfassend ein physiologisch annehmbares Medium und einen Extrakt von Artemia salina, erhalten durch das Verfahren, umfassend die folgenden Schritte: a) Zysten von Artemia salina werden während 30 Minuten bis 6 Stunden in destilliertem Wasser, bei einer Temperatur zwischen 30°C und 75°C und bei einem pH zwischen 4 und 7, rehydriert, b) die Zysten werden anschließend zerkleinert, und c) der so erhaltene Extrakt wird zentrifugiert und filtriert, um die Haut gegen ein Austrocknen zu schützen, das mit Aggressionen aufgrund von kalten thermischen Belastungen verbunden ist, indem das Aufrechterhalten eines physiologischen Gehalts an CIRP (Cold-inducible RNA Binding Protein) gestattet wird.

2. Verwendung nach Anspruch 1, wobei der Extrakt von Artemia salina mit einem oder mehreren physiologisch annehmbaren Lösungsmitteln verdünnt wird, ausgewählt aus Wasser, Glycerin, Ethanol, Propandiol, Butylenglykol, Dipropylenglykol, ethoxylierten oder propoxylierten Diglykolen, cyclischen Polyolen oder jeder Mischung dieser Lösungsmittel.

3. Verwendung nach einem der Ansprüche 1 oder 2, wobei der Extrakt von Artemia salina in einer Konzentration zwischen 0,0001 % und 20 %, bezogen auf das Gesamtgewicht der Zusammensetzung, und vorzugsweise in einer Konzentration zwischen 0,05 % und 5 %, bezogen auf das Gesamtgewicht der Zusammensetzung, vorliegt.

4. Verwendung nach einem der Ansprüche 1 bis 3, wobei die Zusammensetzung außerdem mindestens ein zusätzliches aktives Mittel umfasst, ausgewählt aus Vitamin A, Retinsäure, Retinol, Vitamin B3, Vitamin B5, Vitamin B6, Vitamin B12, Vitamin C, Vitamin E, Vitamin F, Vitamin H, Vitamin K, Vitamin PP, Coenzym Q10, Metalloproteinase-Inhibitoren, Aminosäuren, Carnitin, Carnosin, Taurin, natürlichen oder Synthese-Peptiden, pflanzlichen Peptid-Extrakten, Hefe-Extrakten, Phytosterinen synthetischen oder natürlichen Ursprungs, Salicylsäure, Oligosacchariden, Polysacchariden, Aminozuckern, Polyphenolen, Flavonoiden, Lipiden, Phospholipiden, cyclischem AMP und seinen Derivaten, Aktivatormitteln des Enzyms Adenylatcyclase, Inhibitormitteln des Enzyms Phosphodiesterase, Thein, Theophyllin, Theobromin, Forskolin, Esculin, Inhibitoren von ACE, dem Extrakt von Dioscorea, dem Extrakt von Guarana, dem Extrakt von Efeu, dem Extrakt von Fucus, den Extrakten von Algen, wie Palmata Palmaria, dem hydrolysierten Extrakt von Prunella vulgaris oder dem Extrakt von Holunder.

5. Zusammensetzung zur topischen Anwendung auf mindestens einem Teil der Haut des Körpers oder des Gesichts, wobei die Zusammensetzung ein physiologisch annehmbares Medium und einen Extrakt von Artemia salina umfasst, erhalten durch das Verfahren, umfassend die folgenden Schritte: a) Zysten von Artemia salina werden während 30 Minuten bis 6 Stunden in destilliertem Wasser, bei einer Temperatur zwischen 30°C und 75°C und bei einem pH zwischen 4 und 7, rehydriert, b) die Zysten werden anschließend zerkleinert, und c) der so erhaltene Extrakt wird zentrifugiert und filtriert, zu seiner Verwendung zum Schutz der Haut gegen Reizungen und Risse, die mit Aggressionen aufgrund von kalten thermischen Belastungen verbunden sind, indem das Aufrechterhalten eines physiologischen Gehalts an CIRP (Cold-inducible RNA Binding Protein) gestattet wird.

## Claims

1. Non-therapeutic use for topical application to at least part of the skin of the body or face of a composition comprising a physiologically acceptable medium and an extract of Artemia salina obtained by a method comprising the following steps: a) rehydrating Artemia salina cysts for 30 minutes to 6 hours in distilled water at a temperature between 30°C and 75°C and at a pH between 4 and 7; b) then crushing the cysts; and c) centrifuging and filtering the extract thus obtained, so as to protect the skin from drying out in association with aggressions caused by cold thermal stresses, making it possible to maintain a physiological level of CIRP (Cold-inducible RNA Binding Protein).

2. The use according to claim 1, wherein said Artemia salina extract is diluted in one or more physiologically acceptable solvents, selected from water, glycerol, ethanol, propanediol, butylene glycol, dipropylene glycol, ethoxylated or propoxylated diglycols, cyclic polyols or any mixture of these solvents.

3. The use according to either one of claims 1 or 2, wherein said extract of Artemia salina is present in a concentration between 0.0001% and 20% of the total weight of the composition, and preferably at a concentration between 0.05% and 5% of the total weight of the composition.

4. The use according to any one of claims 1 to 3, wherein the composition further comprises at least one additional active agent selected from vitamin A, retinoic acid, retinol, vitamin B3, vitamin B5, vitamin B6, vitamin B12, vitamin C, vitamin E, vitamin F, vitamin H, vitamin K, vitamin PP, coenzyme Q10, metalloproteinase inhibitors, amino acids, carnitine, carnosine, taurine, natural or synthetic peptides, plant peptide extracts, yeast extracts, phytosterols of synthetic or natural origin, salicylic acid, oligosaccharides, polysaccharides, amino sugars, polyphenols, flavonoids, lipids, phospholipids, cyclic AMP and derivatives thereof, activators of the enzyme adenylyl cyclase, inhibitors of the enzyme phosphodiesterase, theine, theophylline, theobromine, forskolin, esculin, inhibitors of ACE, dioscorea extract, guarana extract, ivy extract, fucus extract, extracts of algae, such as Palmata Palmaria, hydrolysed extract of Prunella vulgaris, or elderberry extract.

5. A composition for topical application to at least part of the skin of the body or face, said composition comprising a physiologically acceptable medium and an extract of Artemia salina obtained by a method comprising the following steps: a) rehydrating Artemia salina cysts for 30 minutes to 6 hours in distilled water at a temperature between 30°C and 75°C and at a pH between 4 and 7; b) then crushing the cysts; and c) centrifuging and filtering the extract thus obtained, for its use in protecting the skin against irritation and cracking in association with aggressions caused by cold thermal stresses, making it possible to maintain a physiological level of CIRBP (Cold-inducible RNA-Binding Protein).
